# EUROPEAN PATENT APPLICATION

(11) **EP 4 212 172 A1**
(43) Date of publication of application: **19.07.2023**
(21) Application number: 21866754.1
(22) Date of filing: 07.09.2021
(51) Int. Cl.: A61K 39/00, A61K 39/385, A61K 39/39, A61P 25/28, A61P 43/00, C07K 7/06, C07K 14/47, A61K 47/64

(54) **IMMUNOGENIC COMPOSITION TARGETING PHOSPHORYLATED TAU PROTEIN**

(30) Priority: 08.09.2020 JP 2020150890
(71) Applicant: OSAKA UNIVERSITY, Suita-shi Osaka 565-0871 (JP)
(72) Inventor: NAKAGAMI Hironori, Suita-shi, Osaka 565-0871 (JP); MORISHITA Ryuichi, Suita-shi, Osaka 565-0871 (JP); TAKEDA Shuko, Suita-shi, Osaka 565-0871 (JP); HAYASHI Hiroki, Suita-shi, Osaka 565-0871 (JP)
(74) Representative: Witte, Weller & Partner Patentanwälte mbB
(86) International application number: PCT/JP2021/032847
(87) International publication number: WO 2022/054795

(57) **Abstract**

The present invention provides an immunogenic composition comprising an antigenic peptide and a carrier protein, wherein the antigenic peptide is capable of eliciting production of an antibody against a phosphorylated tau protein and is a human tau protein fragment containing (1) YSpSPGpSPG (SEQ ID NO: 2), (2) AKpSpTPpTAE (SEQ ID NO: 5), (3) AKpSpTPTAE (SEQ ID NO: 31), (4) AKpSTPpTAE (SEQ ID NO: 32), (5) AKSpTPpTAE (SEQ ID NO: 33), or (6) IVpYKpSPV (SEQ ID NO: 24).

## Description

### TECHNICAL FIELD

The present invention relates to an immunogenic composition targeting a phosphorylated tau protein.

### BACKGROUND ART

Dementia is a condition of abnormal cognitive decline due to brain structural disorder, not a phenomenon such as an age-related memory or mental function decline. Alzheimer's disease is known as a causative disease of dementia. The pathological hallmarks of Alzheimer's disease include senile plaques and neurofibrillary tangles (NFTs). Senile plaques are formed by extra-neuronal deposition of a peptide called amyloid-β. NFTs are fibrillar aggregates of tau protein.

Tau protein, which was discovered as a microtubule-associated protein (MAP), is expressed in neurons and glial cells of the central and peripheral nervous systems and regulates microtubule polymerization and stabilization. Tau protein binds to a variety of proteins as well as microtubules and are involved in various phenomena that occur in the cranial nervous system, such as postnatal brain maturation, regulation of axonal transport and signaling, cellular response to heat stress, and adult neurogenesis. Amyloid-β and inflammation cause abnormal phosphorylation of tau protein. This results in the depolymerization of microtubules, the detachment of tau protein from microtubules, and the oligomerization of tau protein monomers, which further aggregate and fibrillize into NFTs, causing neuronal damage and eventual cell death. In addition, pathological forms of tau protein are extracellularly released or secreted, are internalized into other neurons, and form new aggregates therein. This phenomenon is well known and called "tau protein propagation". In general, as Alzheimer's disease progresses, tau aggregates spread more widely.

Tau protein is known to have numerous phosphorylation sites. These phosphorylation sites can be reportedly classified into three categories: ones found in the brains of Alzheimer's disease patients, ones found in the brains of both Alzheimer's disease patients and healthy individuals, and ones found in the brains of healthy individuals (Non-Patent Literature 1). To date, antibodies against phosphorylated forms of tau protein have been developed and tested therapeutically, but none of them have shown promising high therapeutic efficacy. However, not all of the phosphorylation sites found in the brains of Alzheimer's disease patients have been examined as targets for antibody production. There is hope of finding epitopes that contribute to high therapeutic efficacy, leading to the development of vaccines to inhibit the progression of Alzheimer's disease.

### CITATION LIST

### Non-Patent Literature

Non-Patent Literature 1: Simic G et al., Biomolecules. 2016 Jan 6;6(1):6. doi: 10.3390/biom6010006.

### SUMMARY OF INVENTION

### TECHNICAL PROBLEM

An object of the present invention is to provide an immunogenic composition capable of eliciting production of an antibody against a phosphorylated tau protein. Another object of the present invention is to provide an immunogenic composition capable of eliciting production of an antibody against a pathological hyperphosphorylated tau protein present in the brains of Alzheimer's disease patients.

### SOLUTION TO PROBLEM

The present invention includes the following to achieve the above-mentioned obj ects.
[1] An immunogenic composition comprising an antigenic peptide and a carrier protein, wherein the antigenic peptide is capable of eliciting production of an antibody against a phosphorylated tau protein and is a human tau protein fragment containing
   (1) YSpSPGpSPG (SEQ ID NO: 2),
   (2) AKpSpTPpTAE (SEQ ID NO: 5),
   (3) AKpSpTPTAE (SEQ ID NO: 31),
   (4) AKpSTPpTAE (SEQ ID NO: 32),
   (5) AKSpTPpTAE (SEQ ID NO: 33), or
   (6) IVpYKpSPV (SEQ ID NO: 24).
[2] The composition according to the above [1], wherein the antigenic peptide has at least two phosphorylated amino acids.
[3] The composition according to the above [1] or [2], wherein the antigenic peptide has a length of 10 amino acids or less.
[4] The composition according to the above [1], wherein the antigenic peptide is a phosphorylated peptide of any one of (1) to (6).
[5] The composition according to any one of the above [1] to [4], wherein the phosphorylated tau protein is a pathological hyperphosphorylated tau protein.
[6] The composition according to any one of the above [1] to [5], wherein the carrier protein is keyhole limpet hemocyanin.
[7] The composition according to any one of the above [1] to [6], wherein the composition comprises a conjugate of the antigenic peptide and the carrier protein.
[8] The composition according to any one of the above [1] to [7], further comprising an adjuvant.
[9] The composition according to any one of the above [1] to [8], wherein the composition is for treatment of tauopathy.
[10] The composition according to the above [9], wherein the tauopathy is Alzheimer's disease.
[11] A vaccine for treating tauopathy, comprising the composition according to any one of the above [1] to [8].
[12] The vaccine according to the above [11], wherein the tauopathy is Alzheimer's disease.

### ADVANTAGEOUS EFFECTS OF INVENTION

The present invention provides an immunogenic composition capable of eliciting production of an antibody against a phosphorylated tau protein present in the brains of Alzheimer's disease patients.

### BRIEF DESCRIPTION OF DRAWINGS

Fig. 1 shows the results of the primary screening of 30 phosphorylated tau epitope candidates. In this screening, AJ peptide-conjugated phosphorylated tau epitope candidates were administered to mice, and antibody titers against the epitope candidates were measured.
Fig. 2 shows the results of the secondary screening of the 11 phosphorylated tau epitope candidates selected in the primary screening. In this screening, keyhole limpet hemocyanin (KLH)-conjugated phosphorylated tau epitope candidates were administered to mice, and antibody titers against the epitope candidates were measured.
Fig. 3 shows the results of the tertiary screening of the 8 phosphorylated tau epitope candidates selected in the secondary screening. In this screening, KLH-conjugated phosphorylated tau epitope candidates were administered to mice, and the produced antibodies were examined for the ability to recognize hyperphosphorylated tau protein in brain extracts from PS19 Tg mice (tauopathy model mice), but not tau protein in brain extracts from wild-type mice by western blotting.
Fig. 4 shows the results of the evaluation of the 3 phosphorylated tau epitope candidates selected in the tertiary screening. In this evaluation, KLH-conjugated phosphorylated tau epitope candidates were administered to rabbits, and the produced antibodies were examined for binding to a hyperphosphorylated tau protein having aggregation activity in brain extracts from PS19 Tg mice.
Fig. 5 shows the experimental protocol used in Example 3.
Fig. 6 shows the time-course change in serum neutralizing antibody titer in PS 19 Tg mice and wild-type mice treated with KLH-conjugated phosphorylated tau epitope YAV-5.
Fig. 7 shows the relationship between the serum neutralizing antibody titer and the cerebrospinal fluid (CSF) neutralizing antibody titer in PS19 Tg mice treated with KLH-conjugated phosphorylated tau epitope YAV-5.
Fig. 8 shows the seeding activity in PBS-soluble brain extracts from PS19 Tg mice treated with KLH-conjugated phosphorylated tau epitope YAV-5. Fig. 8A shows representative images of intracellular tau aggregates in tau biosensor cells. Fig. 8B is a graph showing the number of intracellular tau aggregates normalized to the number of cells (DAPI-positive cells).
Fig. 9 shows the amount of tau aggregates in the insoluble fraction of the brains from PS19 Tg mice treated with KLH-conjugated phosphorylated tau epitope YAV-5.
Fig. 10 shows the results of observation of NFT-like lesions in the brains from PS19 Tg mice treated with KLH-conjugated phosphorylated tau epitope YAV-5. Fig. 10A shows representative images of brain histological sections immunostained with anti-AT8 antibody. Fig. 10B shows the numbers of AT8-positive cells counted in the hippocampal regions.
Fig. 11 shows the tau concentration in the cerebrospinal fluid (CSF) from PS19 Tg mice treated with KLH-conjugated phosphorylated tau epitope YAV-5.

### DESCRIPTION OF EMBODIMENTS

The present invention provides an immunogenic composition comprising an antigenic peptide and a carrier protein, wherein the antigenic peptide is capable of eliciting production of an antibody against a phosphorylated tau protein. The antigenic peptide in the immunogenic composition of the present invention may be a human tau protein fragment containing any one or more of the phosphopeptides (1) to (6) as follows.
(1) YSpSPGpSPG (SEQ ID NO: 2)
(2) AKpSpTPpTAE (SEQ ID NO: 5)
(3) AKpSpTPTAE (SEQ ID NO: 31)
(4) AKpSTPpTAE (SEQ ID NO: 32)
(5) AKSpTPpTAE (SEQ ID NO: 33)
(6) IVpYKpSPV (SEQ ID NO: 24)

The human tau gene is located on the long arm of chromosome 17 and consists of 16 exons. There are six human tau protein isoforms due to selective splicing of exon 2, exons 2 and 3, and exon 10. 2N4R (no exon skipping) has a length of 441 amino acids, 1N4R (skipping of exon 3) has a length of 412 amino acids, 0N4R (skipping of exons 2 and 3) has a length of 383 amino acids, 2N3R (skipping of exon 10) has a length of 410 amino acids, 1N3R (skipping exons 3 and 10) has a length of 381 amino acids, and 0N3R (skipping of exons 2, 3, and 10) has a length of 352 amino acids. The amino acid sequence of the human tau protein 2N4R is shown in SEQ ID NO: 34. Skipping of exon 3 results in the deletion of a region of aspartate (D) at position 74 to threonine (T) at position 102 of SEQ ID NO: 34. Skipping of exons 2 and 3 results in the deletion of a region of glutamate (E) at position 45 to threonine (T) at position 102 of SEQ ID NO: 34. Skipping of exon 10 results in the deletion of a region of valine (V) at position 275 to serine (S) at position 305 of SEQ ID NO: 34.

The phosphopeptide (1) is a peptide which is composed of 8 amino acids corresponding to positions 197 to 204 of SEQ ID NO: 34 and has a phosphorylated serine (S) at position 199 and a phosphorylated serine (S) at position 202. The phosphopeptide (2) is a peptide which is composed of 8 amino acids corresponding to positions 66 to 73 of SEQ ID NO: 34 and has a phosphorylated serine (S) at position 68, a phosphorylated threonine (T) at position 69, and a phosphorylated threonine (T) at position 71. The phosphopeptide (3) is a peptide which is composed of 8 amino acids corresponding to positions 66 to 73 of SEQ ID NO: 34 and has a phosphorylated serine (S) at position 68 and a phosphorylated threonine (T) at position 69. The phosphopeptide (4) is a peptide which is composed of 8 amino acids corresponding to positions 66 to 73 of SEQ ID NO: 34 and has a phosphorylated serine (S) at position 68 and a phosphorylated threonine (T) at position 71. The phosphopeptide (5) is a peptide which is composed of 8 amino acids corresponding to positions 66 to 73 of SEQ ID NO: 34 and has a phosphorylated threonine (T) at position 69 and a phosphorylated threonine (T) at position 71. The phosphopeptide (6) is a peptide which is composed of 7 amino acids corresponding to positions 392 to 398 of SEQ ID NO: 34 and has a phosphorylated tyrosine (Y) at position 394 and a phosphorylated serine (S) at position 396.

The antigenic peptide in the immunogenic composition of the present invention may be a human tau protein fragment containing any one of the phosphopeptides (1) to (6) described above. Preferred is a human tau protein fragment containing any one of the phosphopeptides (1), (2) and (6). More preferred is a human tau protein fragment containing the phosphopeptide (1) or (2). The antigenic peptide preferably has at least two phosphorylated amino acids. The antigenic peptide may have 3 or more phosphorylated amino acids, 4 or more phosphorylated amino acids, or 5 or more phosphorylated amino acids.

The number of amino acids in the antigenic peptide is not particularly limited and may be 30 or less, 25 or less, 20 or less, 18 or less, 16 or less, 15 or less, 14 or less, 13 or less, 12 or less, 11 or less, 10 or less. Preferably, the number of amino acids in the antigenic peptide is 10 or less and may be 9 or less, 8 or less, or 7 or less.

The antigenic peptide in the immunogenic composition of the present invention is preferably any one of the phosphopeptides (1) to (6) described above. Particularly, it is preferably any one of the phosphopeptides (1), (2) and (6), and it is preferably the phosphopeptide (1) or (2).

The phosphorylated tau protein that is targeted by the immunogenic composition of the present invention is preferably a pathological hyperphosphorylated tau protein present in the brains of Alzheimer's disease patients. The pathological hyperphosphorylated tau protein present in the brains of Alzheimer's disease patients refers to a hyperphosphorylated human tau protein having so-called seeding activity. The hyperphosphorylated human tau protein having seeding activity is considered to be a hyperphosphorylated human tau protein having the ability to aggregate and propagate.

The carrier protein in the immunogenic composition of the present invention is not particularly limited and can be selected from known carrier proteins that can be used in vaccines. Examples of the known carrier protein include albumin, ovalbumin, keyhole limpet hemocyanin (KLH), *Pseudomonas aeruginosa* exotoxin, tetanus toxin, ricin toxin, diphtheria toxin, cholera toxin, heat-labile enterotoxin, epidermal growth factor, fibroblast growth factor, transferrin, platelet-derived growth factor, poly-L-lysine, poly-L-glutamine, mannose-6-phosphate, and hepatitis B viral core protein. Particularly preferred is keyhole limpet hemocyanin (KLH).

AJ peptide (ELKLIFLHRLKRLRKRLKRK, SEQ ID NO: 35), which was discovered by the present inventors, may also be used as a carrier protein. The AJ peptide contains a T-cell epitope. This is advantageous in that the AJ peptide can elicit the production of the desired antibody without the use of adjuvants (Tenma A et al., FASEB BioAdvances, 2019; 1: 760-772).

The antigenic peptide is preferably connected to the carrier protein to form a conjugate. The antigenic peptide may be connected directly to the carrier protein like a fusion protein, or connected to the carrier protein via a linker (used as a synonym for a spacer). The linker is not particularly limited as long as it is capable of connecting the antigenic peptide and the carrier protein. Examples of the linker include aminocarboxylic acids such as β-aminoalanine, γ-aminobutyric acid, ε-aminocaproic acid, 7-aminoheptanoic acid, 12-aminolauric acid, glutamic acid, and p-aminobenzoic acid. Other examples include L-amino acids, which are present in naturally occurring proteins, and D-isomers thereof. In addition, crosslinkers, such as EMCS (N-(6-maleimidocaproyloxy)succinimide), glutaraldehyde, and Sulfo-GMBS (N-γ-maleimidobutyryl-oxysulfosuccinimide ester), can be used.

When the carrier protein is KLH and the antigenic peptide lacks lysine (K), glutaraldehyde can be used as a preferable linker. Even when the antigenic peptide has lysine, as long as the lysine is located only at the N- or C-terminus of the antigenic peptide, glutaraldehyde can be used as a preferable linker. When the antigenic peptide has lysine at a site other than the N- or C-terminus, it is preferable to introduce cysteine (C) into the N- or C-terminus of the antigenic peptide and/or to use EMCS for connecting the antigenic peptide to the carrier protein. Alternatively, regardless of the sequence of the antigenic peptide, the connection of the antigenic peptide to the carrier protein can be achieved using Sulfo-GMBS after cysteine introduction into the C-terminus of the antigenic peptide.

The order of arrangement of the antigenic peptide and the carrier protein in the conjugate is not particularly limited. The carrier protein and the antigenic peptide may be on the N-terminal and C-terminal sides, respectively. Conversely, the antigenic peptide and the carrier protein may be on the N-terminal and C-terminal sides, respectively. Preferably, the carrier protein and the antigenic peptide may be on the N-terminal and C-terminal sides, respectively.

The conjugate of the antigenic peptide and the carrier protein is preferably acetylated at its N-terminal amino acid. The conjugate is preferably amidated at its C-terminal amino acid. More preferably, the conjugate is acetylated at its N-terminal amino acid and amidated at its C-terminal amino acid.

The immunogenic composition of the present invention may further comprise one or more adjuvants. The adjuvant can be selected as appropriate from known adjuvants. Specific examples of the known adjuvant include aluminum adjuvants (e.g., aluminum salts such as aluminum hydroxide, aluminum phosphate, and aluminum sulfate, or a combination thereof), complete or incomplete Freund's adjuvant, TLR ligands (e.g., CpG, Poly(I:C), Pam3CSK4, etc.), BAY, DC-chol, pcpp, monophosphoryl lipid A, QS-21, cholera toxin, and formylmethionyl peptides. Preferred are aluminum adjuvants, TLR ligands, and a combination thereof. In the immunogenic composition of the present invention comprising an adjuvant, the amount of the adjuvant is not particularly limited and can be selected as appropriate according to the type of adjuvant to be used, etc.

Administration of the immunogenic composition of the present invention to a human results in the production of an antibody against a pathological hyperphosphorylated tau protein, thereby treating tauopathy. Therefore, the immunogenic composition of the present invention is suitable for use as a vaccine for treating tauopathy. Treating tauopathy includes delaying or inhibiting the progression of tauopathy. That is, a vaccine for treating tauopathy comprising the immunogenic composition of the present invention may be referred to as a vaccine for delaying tauopathy progression or a vaccine for inhibiting tauopathy progression.

Tauopathy is a general term for diseases presumably caused mainly by abnormal intracellular accumulation of an excessively phosphorylated and insoluble form of tau protein. Examples of the tauopathy include Alzheimer's disease, Pick disease, progressive supranuclear palsy, corticobasal degeneration, Down syndrome, frontotemporal dementia and parkinsonism linked to chromosome 17 (FTDP-17), argyrophilic grain dementia, NFT-predominant form of senile dementia, and subacute sclerosing panencephalitis. The immunogenic composition of the present invention is preferably used as a vaccine for treating Alzheimer's disease.

The immunogenic composition of the present invention can be administered orally or parenterally. The parenteral administration includes intraperitoneal administration, subcutaneous administration, intracutaneous administration, intramuscular administration, intravenous administration, intranasal administration, transdermal administration, transmucosal administration, sublingual administration, and inhalation administration. Preferred is parenteral administration, and more preferred are intracutaneous administration, subcutaneous administration, and intramuscular administration. For parenteral administration, microneedle injection, non-needle injection, a stamping method, etc. may be employed.

The immunogenic composition of the present invention can be formulated into a dosage form by combining the antigenic peptide capable of eliciting production of an antibody against a phosphorylated tau protein with a carrier protein, a pharmaceutically acceptable carrier, and optionally an additive. Specific examples of the dosage form include oral preparations such as tablets, coated tablets, pills, powders, granules, capsules, solutions, suspensions, and emulsions; and parenteral preparations such as injections, infusions, suppositories, ointments, and patches. The amount of the carrier or the additive to be used is determined as appropriate based on the range of amount conventionally used in the pharmaceutical field. The carrier or the additive that can be used is not particularly limited, and examples include various carriers such as water, physiological saline, other aqueous solvents, and aqueous or oily bases; and various additives such as excipients, binders, pH adjusters, disintegrants, absorption enhancers, lubricants, colorants, corrigents, and fragrances.

Examples of the additive used for solid oral preparations include excipients such as lactose, mannitol, glucose, microcrystalline cellulose, and corn starch; binders such as hydroxypropyl cellulose, polyvinylpyrrolidone, and magnesium aluminometasilicate; dispersants such as corn starch; disintegrants such as calcium carboxymethyl cellulose; lubricants such as magnesium stearate; solubilizing agents such as glutamic acid and aspartic acid; stabilizers; water soluble polymers including celluloses such as hydroxypropyl cellulose, hydroxypropylmethyl cellulose, and methyl cellulose, and synthetic polymers such as polyethylene glycol, polyvinylpyrrolidone, and polyvinyl alcohol; sweeteners such as white sugar, powder sugar, sucrose, fructose, glucose, lactose, reduced malt sugar syrup (maltitol syrup), reduced malt sugar syrup powder (maltitol syrup powder), high-glucose corn syrup, high-fructose corn syrup, honey, sorbitol, maltitol, mannitol, xylitol, erythritol, aspartame, saccharin, and saccharin sodium; and coating agents such as white sugar, gelatin, hydroxypropyl cellulose, and hydroxypropylmethyl cellulose phthalate.

Liquid oral preparations can be prepared by dissolution, suspension, or emulsification in a commonly used diluent. Examples of the diluent include purified water, ethanol, and a mixture thereof. The liquid oral preparation may further contain a wetting agent, a suspending agent, an emulsifier, a sweetener, a flavoring agent, a fragrance, a preservative, a buffering agent, and/or the like.

Examples of the additive used for injections for parenteral administration include isotonizing agents such as sodium chloride, potassium chloride, glycerin, mannitol, sorbitol, boric acid, borax, glucose, and propylene glycol; buffering agents such as a phosphate buffer solution, an acetate buffer solution, a borate buffer solution, a carbonate buffer solution, a citrate buffer solution, a Tris buffer solution, a glutamate buffer solution, and an ε-aminocaproate buffer solution; preservatives such as methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, disodium edetate, boric acid, and borax; thickeners such as hydroxyethyl cellulose, hydroxypropyl cellulose, polyvinyl alcohol, and polyethylene glycol; stabilizers such as sodium hydrogen sulfite, sodium thiosulfate, disodium edetate, sodium citrate, ascorbic acid, and dibutylhydroxytoluene; and pH adjusters such as hydrochloric acid, sodium hydroxide, phosphoric acid, and acetic acid. The injection may further contain an appropriate solubilizer. Examples of the solubilizer include alcohols such as ethanol; polyalcohols such as propylene glycol and polyethylene glycol; and nonionic surfactants such as polysorbate 80, polyoxyethylene hydrogenated castor oil 50, lysolecithin, and Pluronic polyol. Liquid preparations such as injections can be preserved in a frozen state, or in a dried state after water removal by lyophilization, etc. Lyophilized preparations can be reconstituted in distilled water for injection or the like just before use.

The immunogenic composition of the present invention can be administered to any animal (a human or a non-human animal) having an immune system. Examples of the animal include mammals such as humans, monkeys, cattle, horses, pigs, sheep, goats, dogs, cats, guinea pigs, rats, and mice; and birds such as chickens, ducks, and geese. Preferably, the immunogenic composition of the present invention is administered to a human child or adult.

The dosing frequency and interval of the immunogenic composition of the present invention are not particularly limited. For example, the immunogenic composition may be administered once, or multiple times at intervals of about two days to about eight weeks. The dose of the immunogenic composition varies with the subject of administration, the method of administration, etc., but the amount of the antigenic peptide in a dose per administration is preferably about 0.01 µg to about 10 mg, more preferably about 0.1 µg to about 1 mg, and still more preferably about 1 µg to about 0.1 mg.

The present invention includes the following.

A method for treating tauopathy, comprising administering the immunogenic composition of the present invention to an animal.

A method for treating Alzheimer's disease, comprising administering the immunogenic composition of the present invention to an animal.

The immunogenic composition of the present invention for use in treatment of tauopathy.

The immunogenic composition of the present invention for use in treatment of Alzheimer's disease.

Use of the immunogenic composition of the present invention for production of a vaccine or medicament for treating tauopathy.

Use of the immunogenic composition of the present invention for production of a vaccine or medicament for treating Alzheimer's disease.

### EXAMPLES

Hereinafter, the present invention will be described in detail by examples, but the present invention is not limited thereto.

### Example 1: Screening of phosphorylated tau epitopes

### (1) Primary screening

Thirty phosphorylated tau epitopes (YAV-1 to YAV-30, SEQ ID NOs: 1 to 30) of 5 to 9 amino acids, including at least one phosphorylated amino acid, were used for primary screening. The numbers indicating the positions of the phosphorylated amino acids represent the positions of the corresponding amino acids in the amino acid sequence of the 2N4R isoform of human tau protein (SEQ ID NO: 34). Individual phosphorylated tau epitopes were connected to AJ peptide (SEQ ID NO: 35) via ε-aminocaproic acid (ε-Acp) to prepare conjugate peptides (see Table 1, hereinafter referred to as "AJ conjugates"). The AJ conjugates were administered intracutaneously to BALB/c mice (male, 7 weeks old) (500 µg/100 µL/mouse, N=4). The second dose was administered 2 weeks later. After 5 weeks, blood was collected, and serum was separated for antibody titer measurement.

**[Table 1]**

| Phosphorylated tau epitope No. | Structure of AJ conjugate (X=ε-Acp) |
|---|---|
| YAV-1 | Ac-AJP-X-VYKpS(396)PVV-amide |
| YAV-2 | Ac-AJP-X-YSpS(199)PGpS(202)PG-amide |
| YAV-3 | Ac-AJP-X-AGTpY(1 8)GLG-amide |
| YAV-4 | Ac-AJP-X-MVDpS(422)PQL-amide |
| YAV-5 | Ac-AJP-X-AKpS(68)pT(69)PpT(71)AE-amide |
| YAV-6 | Ac-AJP-X-PKpT(181)PPpS(184)pS(185)GE-amide |
| YAV-7 | Ac-AJP-X-KIGpS(356)LDN-amide |
| YAV-8 | Ac-AJP-X-SRpT(212)PpS(214)LP-amide |
| YAV-9 | Ac-AJP-X-RpT(231)PPKpS(235)P-amide |
| YAV-10 | Ac-AJP-X-KpS(258)KIGpS(262)T-amide |
| YAV-11 | Ac-AJP-X-LKEpS(46)PLQ-amide |
| YAV-12 | Ac-AJP-X-DTPpS(113)LED-amide |
| YAV-13 | Ac-AJP-X-GHVpT(123)QAR-amide |
| YAV-14 | Ac-AJP-X-KIApT(153)PRG-amide |
| YAV-15 | Ac-AJP-X-PAKpT(175)PPA-amide |
| YAV-16 | Ac-AJP-X-PPKpS(191)GDR-amide |
| YAV-17 | Ac-AJP-X-NVQpS(289)KCG-amide |
| YAV-18 | Ac-AJP-X-NVSpS(413)TG-amide |
| YAV-19 | Ac-AJP-X-NVpS(412)pS(413)TG-amide |
| YAV-20 | Ac-AJP-X-EVpS(433)ApS(435)LA-amide |
| YAV-21 | Ac-AJP-X-PGpT(205)PG-amide |
| YAV-22 | Ac-AJP-X-GpS(208)RpS(210)R-amide |
| YAV-23 | Ac-AJP-X-LPpT(217)PP-amide |
| YAV-24 | Ac-AJP-X-IVp Y(3 94)KpS(3 96)P V-amide |
| YAV-25 | Ac-AJP-X-VVpS(400)GD-amide |
| YAV-26 | Ac-AJP-X-GDpT(403)pS(404)PR-amide |
| YAV-27 | Ac-AJP-X-HLpS(409)NV-amide |
| YAV-28 | Ac-AJP-X-TGpS(416)ID-amide |
| YAV-29 | Ac-AJP-X-LApT(427)LA-amide |
| YAV-30 | Ac-AJP-X-GGpY(29)pT(30)MH-amide |

Antibody titers were measured by ELISA. 96-well plates were coated with 10 µg/mL of each AJ conjugate and incubated at 4°C overnight. The plates were blocked with PBS containing 5% skim milk for 2 hours. Diluted sera were added and incubated at 4°C overnight. The plates were washed with PBS containing 0.05% Tween 20. HRP-labeled anti-mouse IgG antibody was added and incubated for 3 hours. 3,3',5,5'-tetramethylbenzine (TMB) was added for HRP-mediated chromogenic reaction. After 30 minutes, sulfuric acid was added to stop the reaction. Absorbances were measured at a wavelength of 450 nm using a microplate reader (brand name: iMark, Bio-Rad).

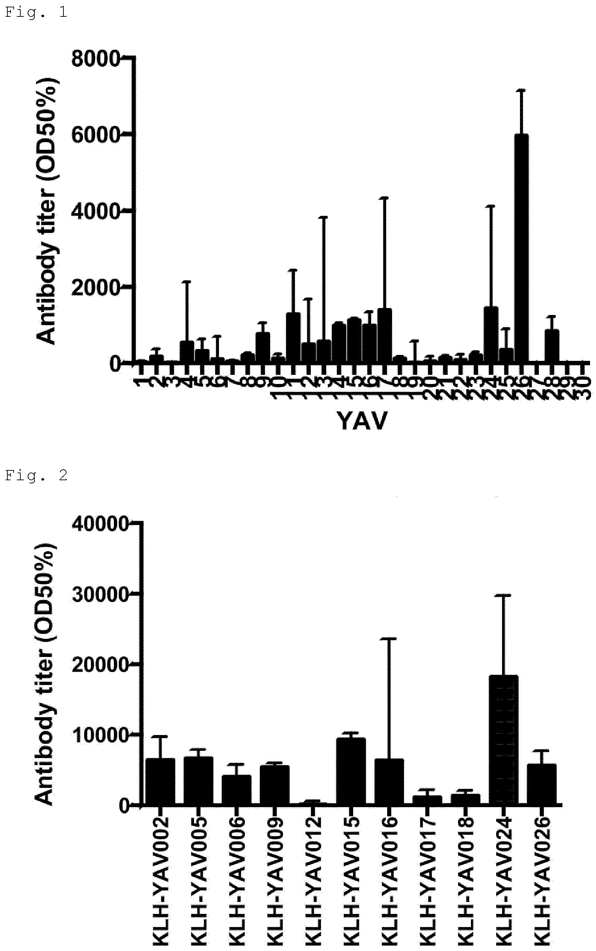

The results are shown in Fig. 1. The antibody titer is expressed as a half maximum value. The half maximum value is a value of a dilution factor at which the absorbance of the serum sample is half of the maximum absorbance measured in a measurement device. The half maximum value can be obtained from a sigmoid curve created by plotting serum dilution factors against measured absorbances. Eleven phosphorylated tau epitopes (YAV-2, 5, 6, 9, 12, 15, 16, 17, 18, 24, and 26) were selected from the epitopes in the AJ conjugates used for the primary screening because they elicited somewhat higher antibody titers.

### (2) Secondary screening

The 11 individual phosphorylated tau epitopes selected in the primary screening were connected to keyhole limpet hemocyanin (KLH) to form conjugate peptides (hereinafter referred to as "KLH conjugates"). The KLH conjugates were administered twice to BALB/c mice in the same manner as in the primary screening. Five weeks after the first administration, blood was collected, and serum was separated for antibody titer measurement and western blotting. Each phosphorylated tau epitope and KLH were connected via glutaraldehyde or EMCS, whichever was appropriate for the sequence of the phosphorylated tau epitope to be used.

Antibody titers were measured by ELISA. Antibody titers were measured in the same manner as in the primary screening, except that 96-well plates were coated with the KLH conjugates. The results are shown in Fig. 2. The antibody titer is expressed as a half maximum value. Eight of the 11 phosphorylated tau epitopes (YAV-2, 5, 6, 9, 15, 16, 24, and 26) elicited higher antibody titers.

### (3) Tertiary screening

The brains of 4-month-old PS19 Tg mice (Jackson Laboratory) or wild-type mice (WT littermates of PS19 Tg mice (genetic background: C57B6xC3H)) were harvested and homogenized in cold PBS containing protease inhibitors (Roche). The homogenates were sonicated and centrifuged at 15,000 × g for 5 minutes. The supernatants were collected and stored at -80°C. PS19 Tg mice are tauopathy model mice generated by introduction of a gene encoding a P301S-1N4R isoform of human tau protein downstream of the prion promoter in TG mice. These mice have been shown to have brain atrophy and intraneuronal tau aggregate formation at 6 to 8 months of age (see https://www.alzforum.org/research-models/tau-p301s-line-ps19).

The brain extracts from wild-type mice (2 µg each), the brain extracts from PS19 Tg mice (2 µg each), and recombinant tau protein (100 ng) were separately boiled at 95°C for 5 minutes and subjected to SDS-PAGE for protein separation. The separated proteins were transferred to PVDF membranes. The membranes were blocked with 5% skim milk for 1 hour. Sera from mice immunized with the KLH conjugates of YAV-2, 5, 6, 9, 15, 16, 24, and 26, anti-tau antibody (Tau-5), and anti-phosphorylated tau antibody (p396) were used for the primary antibodies. The membranes were incubated with the sera or antibodies at 4°C overnight. After washing with PBS containing 0.05% Tween 20, the membranes were incubated with HRP-labeled anti-rabbit IgG antibody or HRP-labeled anti-mouse IgG antibody at room temperature for 1 hour. The antibody-protein binding was assessed by chemiluminescence (Chemi-Lumi One L, Nakalai Tesque).

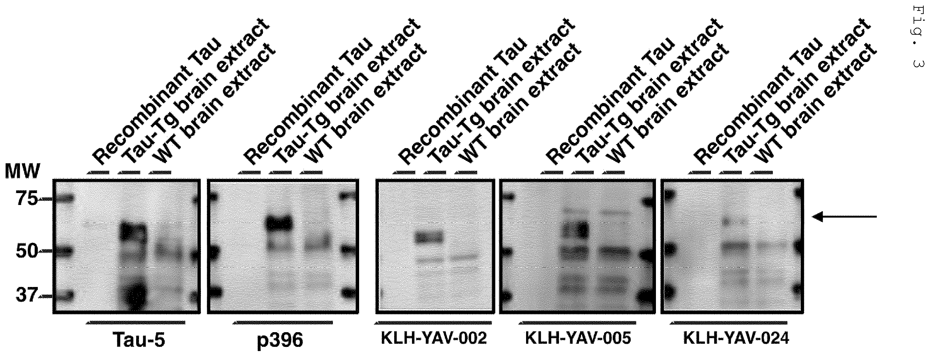

The results are shown in Fig. 3. The sera from mice immunized with YAV-2, YAV-5, or YAV-24 recognized only hyperphosphorylated tau in the brain extracts from PS19 Tg mice, but not tau in the brain extracts from wild-type mice.

### Example 2: Evaluation of the binding of antibodies produced against phosphorylated tau epitopes to a hyperphosphorylated tau protein having seeding activity

Rabbits were immunized with the KLH conjugates of YAV-2, YAV-5, and YAV-24, and phosphorylated tau epitope-specific antibodies were purified. Briefly, each KLH conjugate was administered with Freund's adjuvant to rabbits four times every other week. After checking the antibody titer, blood was collected, serum was separated, and the produced antibody was purified using an epitope-specific affinity column.

The obtained three purified rabbit antibodies were evaluated in comparison with control IgG and anti-tau antibody (Tau-5) as negative controls, and anti-phosphorylated tau antibody (p396) as a positive control. The antibodies were attached to dynabeads protein G (Thermo Fisher) and incubated with brain extracts from PS19 Tg mice at room temperature for 10 minutes. The antibody-bound tau was removed by immunoprecipitation. The supernatants were collected and used as samples. HEK293 cells expressing CFP-/YFP-TauRD (P301S) (Proc Natl Acad Sci USA. 2014 Oct 14;111(41):E4376-4385. doi: 10.1073/pnas.1411649111., Nat Commun. 2015 Oct 13;6:8490. doi: 10.1038/ncomms9490.) were plated in 384-well plates. The following day, the samples were added to the cells with Lipofectamine 2000 (Life Technologies). The cells were fixed with 4% paraformaldehyde (PFA) and subjected to nuclear staining with Hoechst. Confocal microscopy images were obtained via a FRET channel (excited with a 458-nm laser, and fluorescence was captured with a 500-550-nm filter). Tau aggregation was quantified based on fluorescence intensity and normalized to the number of cells.

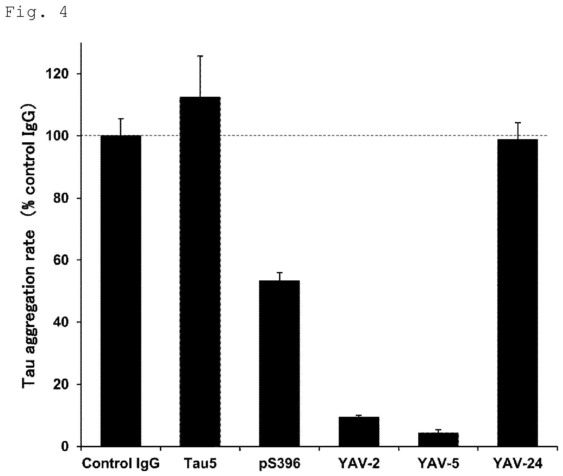

The results are shown in Fig. 4. The seeding activity is represented as the tau aggregation rate for treatment with each antibody, with the tau aggregation rate for treatment with control IgG set at 100%. The aggregation rate for treatment with the antibody produced against YAV-2 or YAV-5 was remarkably low, indicating that both the antibodies are capable of removing a hyperphosphorylated tau protein having seeding activity.

### Example 3: Evaluation of efficacy of a phosphorylated tau epitope as a vaccine

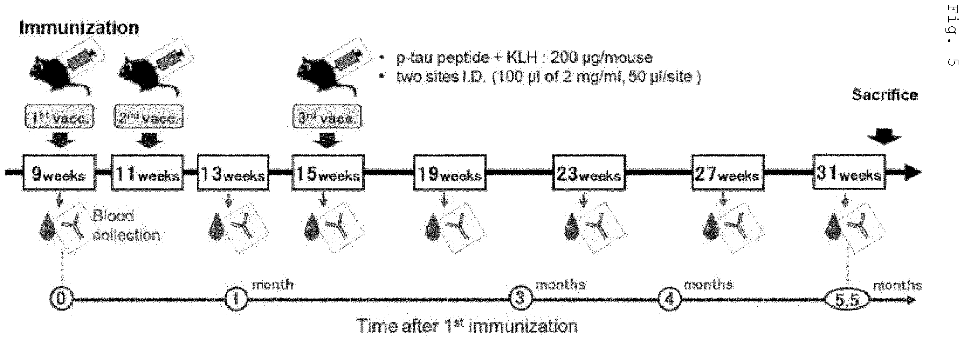

The KLH conjugate of YAV-5 was administered intracutaneously to 9-week-old PS19 Tg mice (Jackson Laboratory) or wild-type mice (WT littermates of PS19 Tg mice (genetic background: C57B6xC3H)). Each mouse received 200 µg of the KLH conjugate at two separate sites (100 µg/50 µL/site). Control IgG was administered as a control. The second and third doses were administered at 11 and 15 weeks of age, respectively. Blood samples were collected at 9, 13, 15, 19, 23, 27, and 31 weeks of age for antibody titer measurement. At 31 weeks of age, the mice were euthanized to harvest brains and cerebrospinal fluids for biochemical and histological analyses. The experimental protocol is shown in Fig. 5.

### (1) Serum neutralizing antibody titer against YAV-5

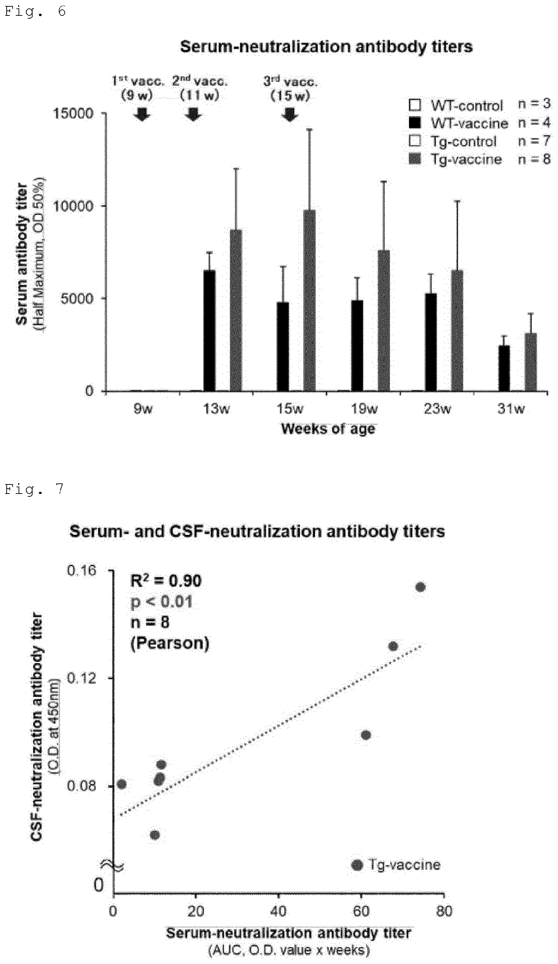

Serum neutralizing antibody titers against YAV-5 were measured by ELISA (see Example 1). The change in neutralizing antibody titer over the period of the first administration (9 weeks of age) to 31 weeks of age is shown in Fig. 6. The antibody titer is expressed as a half maximum value. The administration of the KLH conjugate of YAV-5 strongly elicited the production of a neutralizing antibody against YAV-5 in both PS19 Tg mice and wild-type mice. After the third dose administration at 15 weeks of age, the antibody titer in PS19 Tg mice slightly decreased, but remained at a very high level until 31 weeks of age.

### (2) Relationship between serum neutralizing antibody titer and cerebrospinal fluid (CSF) neutralizing antibody titer

CSF antibody titers were measured by ELISA in the same manner as in the measurement of serum neutralizing antibody titers. The relationship between the serum neutralizing antibody titer and the CSF neutralizing antibody titer is shown in Fig. 7. In Fig. 7, the serum neutralizing antibody titer is expressed as an area under the curve (AUC) value calculated based on the antibody titers measured at 9, 13, 15, 19, 23 and 31 weeks of age. The CSF neutralizing antibody titer is expressed as an OD value measured by ELISA at 31 weeks of age. Both neutralizing antibody titers showed a statistically significant positive correlation (R2=0.90, p<0.05, n=8, Pearson), indicating that a peripherally produced neutralizing antibody against p-tau enters the central nervous system in a dose-dependent manner.

### (3) Effect on tau species involved in tau propagation

The present inventors previously reported that high-molecular-weight phosphorylated tau, which is involved in tau propagation, was detected in a PBS-soluble fraction of brain extracts (Nature communications 6:8490, The American journal of pathology 187(6):1399-1412, PloS one 12(5):e0177914, Nature medicine 26(8):1256-1263). In this present study, the present inventors prepared PBS-soluble brain extracts from brains harvested from 31-week-old mice and evaluated tau seeding activity using tau biosensor cells (see Takeda et al., Nature communications 6:8490). The tau biosensor cells were HEK293 cells stably expressing CFP-/YFP-TauRD(P301S) (Holmes, B. B. et al., Proc. Natl Acad. Sci. USA 111, E4376-E4385 (2014)). The cells were purchased from ATCC. The tau biosensor cells were plated in 96-well PDL-coated plates (3 × 10⁴ cells/well) and cultured overnight. The PBS-soluble brain extract was added to Opti-MEM (Life Technologies) containing Lipofectamine 2000 (Life Technologies), and the mixture was added to culture medium. The tau biosensor cells were cultured in this medium for 48 hours. The cells were fixed with 4% paraformaldehyde (PFA), and the number of tau aggregate-positive cells was counted using flow cytometry (FRET channel). For statistical analysis, Welch's t-test was used.

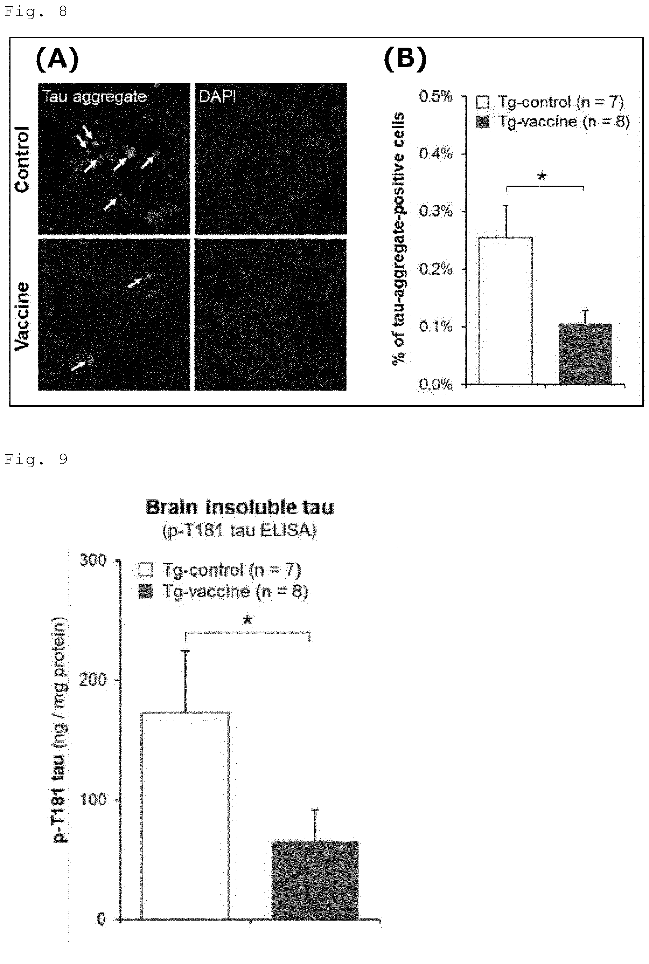

The results are shown in Fig. 8. Fig. 8A shows representative images of intracellular tau aggregates in the tau biosensor cells. White arrows indicate intracellular tau aggregates. Fig. 8B is a graph showing the number of intracellular tau aggregates normalized to the number of cells (DAPI-positive cells). The administration of the KLH conjugate of YAV-5 resulted in an approximately 60% reduction in tau seeding activity in the PS19 Tg mouse brain (∗p<0.05). These results indicate that the antibody against YAV-5 reduced the amount and activity of the tau species involved in propagation.

### (4) Effect on tau aggregates in brain insoluble fraction

Each mouse brain was homogenized in a 5-fold volume of PBS (containing protease inhibitors). The homogenate was centrifuged (10,000 g, 4°C, 15 minutes), and the supernatant (PBS-soluble fraction) was removed. The pellet was incubated with 1% sarcosyl at 37°C for 30 minutes and sonicated for 20 seconds. After centrifugation at 100,000 g at 4°C for 30 minutes, the supernatant was removed, and the residual pellet was solubilized with 100 µL of 8 M urea. After centrifugation at 100,000 g at 4°C for 30 minutes, the supernatant was used as a brain insoluble fraction for measurement. The amount of phosphorylated tau (T181) in the fraction was measured using an ELISA kit (Thermo, KHO0631). The tau concentration was normalized to the total protein concentration in the fraction. For statistical analysis, Mann-Whitney's U test was used. The results are shown in Fig. 9. The administration of the KLH conjugate of YAV-5 resulted in an approximately 60% or greater reduction in the amount of insoluble phosphorylated tau in the PS19 Tg mouse brain (∗p<0.05).

### (5) Effect on NFT-like lesions in the brain

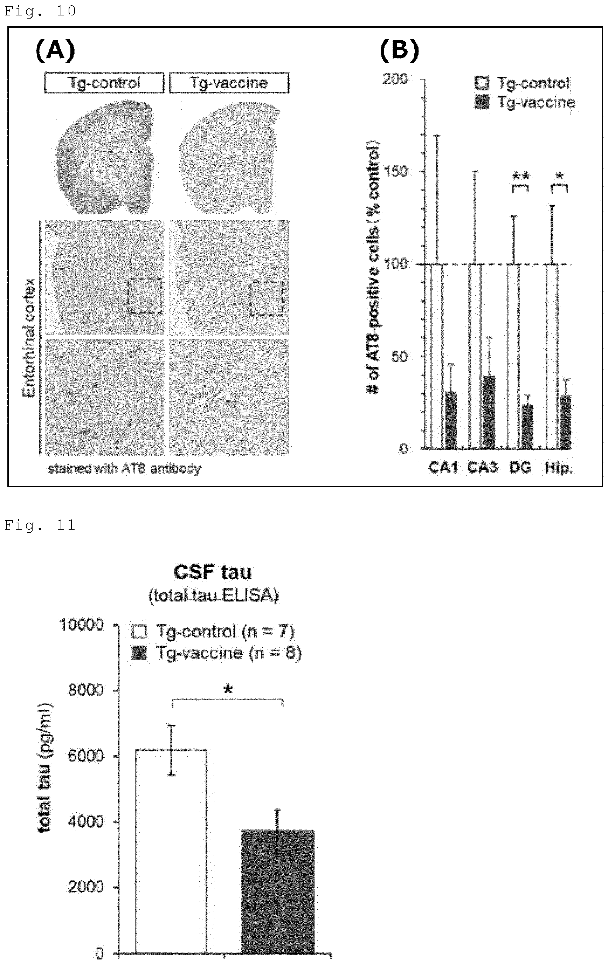

Brain histological sections were prepared according to the method described in Takeda et al. (Nature communications 6:8490). The sections were immunostained with an anti-AT8 antibody that recognizes a tau having a phosphorylated serine 202 and a phosphorylated threonine 205. The number of AT8-positive cells in the hippocampal regions was also counted. For statistical analysis, student's t-test was used. The results are shown in Fig. 10. Fig. 10A shows representative images of immunostaining with the anti-AT8 antibody. The upper panels show the whole brain, the middle panels show the entorhinal cortex, and the lower panels are enlarged views of the dotted frames in the middle panels. Fig. 10B shows the number of AT8-positive cells counted in the hippocampal regions. DG stands for dentate gyrus, and Hip. stands for the whole hippocampus (including CA1, CA3, and DG). The administration of the KLH conjugate of YAV-5 resulted in a significant reduction in AT8-positive NFT-like lesions in the whole brain, including the entorhinal cortex, of PS19 Tg mice. In addition, the administration of the KLH conjugate of YAV-5 resulted in a significant reduction in the number of AT8-positive cells in the dentate gyrus and the whole hippocampus of PS19 Tg mice (∗p<0.05, ∗∗p<0.01).

### (6) Effect on tau concentration in cerebrospinal fluid (CSF)

The tau concentrations in the CSF were compared using a highly sensitive ELISA kit for total tau. Five microliters of mouse spinal fluid collected from the cisterna magna was diluted 10-fold with the buffer contained in the ELISA kit before measurement. ELISA was performed using a Tau (Total) Human ELISA Kit (Thermo, KHB0041). For statistical analysis, student's t-test was used. The results are shown in Fig. 11. The administration of the KLH conjugate of YAV-5 resulted in an approximately 60% reduction in the tau concentration in the PS19 Tg mouse CSF (∗p<0.05). These results indicate that the tau concentration in the CSF can be used as a biomarker to monitor vaccine efficacy against tau lesions in the brain.

The present invention is not limited to particular embodiments and examples described above, and various modifications can be made within the scope of the appended claims. Other embodiments provided by appropriately combining technical means disclosed in separate embodiments of the present invention are also within the technical scope of the present invention. All the academic publications and patent literature cited in the description are incorporated herein by reference.

## Claims

1. An immunogenic composition comprising an antigenic peptide and a carrier protein, wherein the antigenic peptide is capable of eliciting production of an antibody against a phosphorylated tau protein and is a human tau protein fragment containing
(1) YSpSPGpSPG (SEQ ID NO: 2),
(2) AKpSpTPpTAE (SEQ ID NO: 5),
(3) AKpSpTPTAE (SEQ ID NO: 31),
(4) AKpSTPpTAE (SEQ ID NO: 32),
(5) AKSpTPpTAE (SEQ ID NO: 33), or
(6) IVpYKpSPV (SEQ ID NO: 24).

2. The composition according to claim 1, wherein the antigenic peptide has at least two phosphorylated amino acids.

3. The composition according to claim 1 or 2, wherein the antigenic peptide has a length of 10 amino acids or less.

4. The composition according to claim 1, wherein the antigenic peptide is a phosphorylated peptide of any one of (1) to (6).

5. The composition according to any one of claims 1 to 4, wherein the phosphorylated tau protein is a pathological hyperphosphorylated tau protein.

6. The composition according to any one of claims 1 to 5, wherein the carrier protein is keyhole limpet hemocyanin.

7. The composition according to any one of claims 1 to 6, wherein the composition comprises a conjugate of the antigenic peptide and the carrier protein.

8. The composition according to any one of claims 1 to 7, further comprising an adjuvant.

9. The composition according to any one of claims 1 to 8, wherein the composition is for treatment of tauopathy.

10. The composition according to claim 9, wherein the tauopathy is Alzheimer's disease.

11. A vaccine for treating tauopathy, comprising the composition according to any one of claims 1 to 8.

12. The vaccine according to claim 11, wherein the tauopathy is Alzheimer's disease.
